# EUROPEAN PATENT APPLICATION

(11) **EP 4 564 209 A1**
(43) Date of publication of application: **04.06.2025**
(21) Application number: 23217595.0
(22) Date of filing: 18.12.2023
(51) Int. Cl.: G06F 21/57, G06F 21/62, G16H 40/40

(54) **SYSTEMS AND METHODS TO ADVERTISE UNUSED SECURITY FEATURES TO STAKEHOLDERS**

(30) Priority: 29.11.2023 US 202363603770 P
(71) Applicant: Koninklijke Philips N.V., 5656 AG Eindhoven (NL)
(72) Inventor: KOSTER, Robert Paul, Eindhoven (NL)
(74) Representative: Philips Intellectual Property & Standards

(57) **Abstract**

A non-transitory computer readable medium **(16)** stores instructions readable and executable by at least one electronic processor **(14)** to perform a method **(100)** of promoting unused or modified security features to a customer of a medical device **(10).** The method includes detecting, from log data **(11)** of the medical device, an unused or modified security feature of the medical device; determining a contact to whom a notification **(26)** of the unused security feature should be sent; and sending the notification to the determined contact indicative of information of the unused security feature.

## Description

### FIELD OF THE INVENTION

The following relates generally to the medical device maintenance arts, medical device security arts, and related arts.

### BACKGROUND OF THE INVENTION

Many medical devices are computerized. Some examples include medical imaging devices such as magnetic resonance imaging (MRI) scanners, computed tomography (CT) scanners, positron emission tomography (PET) scanners, ultrasound imaging systems, and so forth; image-guided therapy (IGT) systems employing such medical imaging devices; patient monitors, mechanical respirators, and so forth. A computerized medical device should employ appropriate data security measures for numerous reasons, such as to protect sensitive patient medical data acquired or processed by the medical device, to ensure unauthorized personnel do not operate (or interfere with the operation of) the medical device, to ensure the integrity and safe operation of the medical device, and to ensure the medical device does not provide an entry point for unauthorized access to a hospital electronic data network to which the medical device is connected.

Medical device manufacturers enhance security of computerized medical devices by including or adding security features, e.g., encrypted communication, and changing default configuration to be default-secure, e.g., force password-based logins. These features may be included with new medical devices but may also be added to existing medical devices by means of a software update. Such software updates can be pushed via the Internet and the hospital electronic data network.

Medical devices can be protected by various security features, such as: a login password (versus a kiosk mode where anyone can use the imaging device); a password strength requirements (e.g., both upper case and lower case letters, number, special character, minimum password length, et cetera); multifactor authentication (MFA); image encryption; antivirus protection; use of encrypted data transfer via the hospital electronic data network; and so forth. However, these security features often present a tradeoff between security and ease-of-use. For example, employing MFA improves security, but requires users to perform an additional authentication step such as utilizing a dedicated hardware authentication device (e.g., an authentication key) or providing the additional authentication via a cellphone authenticator application program ("app") or text message. In the case of a lost authenticator device or cellphone a user may be unable to access the medical system. In an emergency room setting, for example, it may be preferable to forego MFA to ensure ready access to a critical medical device in an emergency situation. Similarly, certain types of data encryption may be incompatible with some hospital electronic data network configurations. Furthermore, some security features may require customer configuration. Hence, some medical device security features may be implemented in an "opt-in" fashion, in which the customer must activate or turn on (and possibly configure) the security feature. In practice, customers often do not activate many of these security features, e.g., due to lack of awareness of availability of the security feature, or because the customer does not recognize the value of the security feature, or because the customer prefers convenience over the added security provided by the security feature. Not turning on a security feature may be appropriate for a particular customer use case (for example, not turning on MFA in an emergency room or other critical care setting may be acceptable since the emergency room is occupied at all times so that unauthorized use is unlikely, and immediate access is more valuable than the added security in a medical emergency situation), but often unused security features are due to customer ignorance of their availability and/or value.

Furthermore, a given medical device may be under shared control of numerous customer employees, such as the radiology department manager, hospital information technology (IT) personnel, imaging technologists or other operators who actually perform the medical imaging or other medical use, a biomedical equipment technician (biomed) who maintains the imaging device, and/or so forth. This division of "ownership" of the medical device can result in no individual employee proactively acting to activate a particular security feature.

A security breach can produce significant damage such as adversely impacting patient privacy, compromising the hospital electronic data network, or enabling unauthorized use of the medical device. A security breach can also adversely impact the reputation of the vendor, even if the vendor provided an "opt in" security feature that would have prevented the security breach had it been activated. Hence, there is benefit to the customer, the imaging device vendor, and patients in ensuring the customer maximally utilizes available security features of the medical device where appropriate.

The following discloses certain improvements to overcome these problems and others.

### SUMMARY OF THE INVENTION

In one aspect, a non-transitory computer readable medium stores instructions readable and executable by at least one electronic processor to perform a method of promoting unused or modified security features to a customer of a medical device. The method includes detecting, from log data of the medical device, an unused or modified security feature of the medical device; determining a contact to whom a notification of the unused security feature should be sent; and sending the notification to the determined contact indicative of information of the unused security feature.

In another aspect, a non-transitory computer readable medium stores instructions readable and executable by at least one electronic processor to perform a method of promoting unused or modified security features to a customer of a medical device. The method includes detecting, from log data of the medical device, an unused security feature of the medical device; analyzing a list of a plurality of contacts to whom a notification of the unused security feature should be sent; determining information about each contact in the list; selecting a targeted contact based on the determined information; and sending the notification to the selected contact indicative of information of the unused security feature.

In another aspect, a method of promoting unused security features to a customer of a medical device includes detecting, from log data of the medical device, unused security features of the medical device; determining a contact to whom a notification of the unused security features should be sent; generating the notification by aggregating information related to multiple detected unused security features; and sending the notification to the determined contact indicative of information of the unused security feature.

One advantage resides in highlighting unused security features in a medical system.

Another advantage resides in informing stakeholders of a medical device of unused security features.

Another advantage resides in implementing unused security features in a medical system. A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
Fig. 1 diagrammatically shows an illustrative system promoting unused security features to a customer of a medical device in accordance with the present disclosure.
Fig. 2 diagrammatically shows an illustrative system promoting unused security features to a customer of a medical imaging device in accordance with the present disclosure.

### DETAILED DESCRIPTION OF EMBODIMENTS

The following discloses a system for automatically promoting unused security features of a medical device to the customer. To this end, log data of the medical device is analyzed to detect unused security features. This could be direct, e.g., if user login is enforced then each login may be recorded as a log entry. Some detection of unused security features from the log data may be indirect - for example, an unused image encryption feature may be indirectly detected based on the log data not including entries corresponding to loading of public key certificates.

Upon detection of an unused security feature, a security feature usage advertiser determines to whom the notice of unused security feature should be sent. This determination can be made, for example, based on a registry of contacts maintained at the imaging device vendor, or from log file entries if they include identification of imaging technologists, biomeds, or so forth. From this generated list of customer employees (or, more generally, customer contacts, where a customer contact could be an employee under applicable labor law regulations or could be another type of customer contact such as a contracted management person or firm or the like), a targeted contact is selected based on the employee titles or other such information. In another nonlimiting illustrative approach, the log data could be analyzed to determine the most frequent user of the medical device (e.g., based on logged in user information in the log data) and the most frequent user then selected as the targeted customer contact.

The disclosed system then pushes an advertisement or notification of the security feature to the targeted customer contact at a suitable time and via a suitable pathway. For example, if the targeted contact is the radiology department head who is very busy then a suitable pathway may be an email which can be read at any time. If the targeted contact is the biomed then the pathway may be via a message pushed to the imaging device controller when it is in maintenance mode. In some embodiments, the notification can include providing risk factors and a risk type associated with the security feature that is not used (for example, describing potential security breaches that could be prevented by the security feature or specific real-world examples of such breaches if available).

In some embodiments, the advertisement can be constructed by automatically populating a template advertisement with customer-specific information. Optionally, the template may include information such as an indication (e.g., a textual description or a graphic or the like) of the benefits of the unused security feature and/or risks of not activating the unused security feature.

If there are multiple unused security features, then these may be aggregated and pushed in a single message to reduce the number of messages sent to the targeted contact.

An optional further aspect is monitoring the impact of the advertisement, i.e., whether or not the unused or modified security feature is actually activated in response to the advertisement. If the advertisement is not effective, then the system may select a different customer contact to target. Based on feedback as to which customer contact targeting is successful, the algorithm for selection of the targeted customer contact can be updated accordingly. Moreover, the feedback can analyze trends to create new templates or new risk factors. Additionally or alternatively, if there are two or more template advertisements available then a different template advertisement can be populated and sent, which might be more effective for convincing a given customer contact to activate the security feature.

In some embodiments, the transmission of advertisements is recorded in a database to document that the customer has been notified of the unused security features.

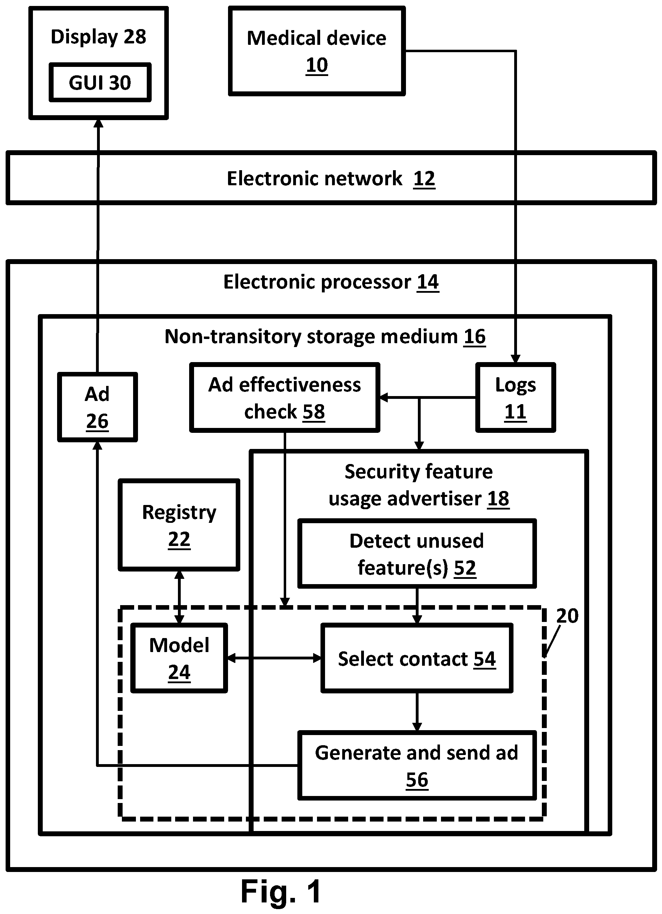

With reference to Fig. 1, an illustrative medical device **10** being monitored is diagrammatically shown. By way of some non-limiting illustrative examples, the medical device **10** being monitored may be a medical imaging device such as a magnetic resonance imaging (MRI) scanner, a computed tomography (CT) scanner, a positron emission tomography (PET) scanner, a gamma camera for performing single photon emission computed tomography (SPECT), an interventional radiology (IR) device, or so forth; or may be another type of medical device such as an image guided therapy (IGT) system, a mechanical ventilator, a multifunction patient monitor, a radiation therapy device, or so forth. The medical device **10** is computerized and may be connected to an electronic network.

As shown in Fig. 1, the medical device **10** transfers log data **11** via an electronic network **12** (e.g., the Internet, a local area network, and so forth - not shown) to a server computer, cloud computing resource, or other electronic processor **14.** The electronic processor **14** includes or is operatively connected with a non-transitory storage medium **16** storing instructions readable and executable by the electronic processor **14** to implement one or more processing modules. Note that the medical device **10** may comprise multiple components: for example, a magnetic resonance (MR) imaging device may include the MR scanner (including the main magnet, gradient coils, radio frequency coils, and so forth) located in the magnet room and an MR scanner controller comprising the electronic processor **14** which is located in a separate, adjacent control room. In this example, the medical device **10** is considered to include both the MR scanner and the MR scanner controller. As another example, the medical device **10** could be an image guided therapy (IGT) system including a medical imaging device and hardware for performing an intravascular procedure or other medical procedure under guidance of the medical imaging device.

The medical device **10** is configured to generate the log data (e.g., logs or log files) **11** of a configuration of the medical device and the use of security features of the medical device **10.** For example, MR scanner, CT scanners, and other complex medical imaging systems automatically generate many gigabytes of log data comprising entries indicating diverse information such as the imaging examinations performed, outputs of sensors, any automatically generated alarms, and so forth. The logs **11** may include system and application event logs that contain events that are indicative for security features being used or not used. Security features can include, for example, disk encryption, software whitelisting, DICOM secure communication, login method, etc. The log data **11** are uploaded to the server **14,** which is controlled by the vendor of the medical device **10.** For example, the server **14** may be owned by the vendor, or may be a cloud computing virtual machine (VM) leased by the vendor. The vendor may use the log data **11** for various purposes. For example, the log data **11** may be consulted by remote service engineers in diagnosing a problem with the medical device **10** reported by the customer. The log data **11** may also be analysed automatically by failure prediction models to proactively predict failure of certain components to enable preventative maintenance to be performed. In this way, downtime of the medical device **10** may be limited and/or scheduled at the customers' convenience. Additionally, as disclosed herein, the log data **11** is analysed to detect unused security features.

As shown in Fig. 1, to this end the processing module(s) can include a security feature usage advertiser **18** operative to analyze the log files **11** to determine unused security features of the medical device **10.** To do so, the security feature usage advertiser **18** is configured to check for events of the medical device **10** indicative of a security feature being used and/or events indicative of a security feature not being used. For example, for secure DICOM, loading of public key certificates may be indicative of non-use, whereas for disk encryption starting disk at encryption services at bootup may be indicative of non-use. The log data **11** may also be analyzed to detect that password-secured logon or MFA security features are not being used, based on log entries related to user's logging into the medical device **10.** The security feature usage analyzer **18** may in this way detect a single unused security feature, or multiple unused security features, if present.

The security feature usage advertiser **18** is further configured to implement a person-of-interest oracle **20** to determine if and how to advertise the unused security feature to a hospital stakeholder (i.e., customer contact). The oracle **20** is configured to determine potential customer contacts from a registry **22.** The registry **22** contains information on customer contacts. For example, the registry **22** may be set up for the customer at the time the customer purchases the medical device **10,** as the paperwork or forms filled out for such a purchase typically include titles, contact information, and the like for one or more customer representatives who serve as customer contacts via which the vendor can contact the customer.

To determine the customer contacts who are candidates to be contacted about the unused security feature detected by the operation **52,** the oracle **20** is configured to select a customer contact from the registry **22.** For this, the oracle **20** may lookup information in the registry **22** and use as-is, or alternatively may implement a machine learning (ML) model **24** to select the most promising customer contact. For example, the ML model **24** may be trained on past historical data indicating which contact roles have in the past been most responsive to notification of an unused security feature by implementing the feature. The model **24** then uses as input data the unused security feature and contact roles listed in the registry **22** to select the targeted contact.

The security feature usage advertiser **18** then prepares an advertisement or notification **26** in an operation **56.** For example, the operation **56** may populate a template advertisement or notification with customer-specific information and/or information about or directed to the specific targeted customer contact. The notification **26** contains information about the device **10** and the unused security feature(s). In some embodiments, the notification **26** can include providing risk factors and a risk type associated with the security feature that is not used Optionally, the notification **26** includes further information providing motivation to implement the unused security feature (for example, describing potential security breaches that could be prevented by the security feature or specific real-world examples of such breaches if available). Optionally, the notification **26** includes instructions on how to activate the currently unused security feature. The security feature usage advertiser **18** communicates the notification **26** to the selected targeted customer contact via a display device **28** presenting a graphical user interface (GU) **30** on which the notification **26** is displayed.

Depending on who the targeted customer contact is, the display device **28** could be the contact's work computer, cellphone, or other device on which the contact reads his or her email, text messages, or other communications, by way of nonlimiting illustrative example. The notification **26** can be transmitted using a communication channel such as e-mail, or an intermediate means such as the medical device **10** or a web portal used by the targeted customer contact. In another example, the display device **28** could be the electronic controller of the medical device **10** itself, and the notification **26** is displayed to the targeted customer contact when he or she logs onto the medical device **10.** In yet another example, if the targeted customer contact is a biomedical engineer, information technology (IT) person, or the like, then the notification **26** can be presented on the electronic controller of the medical device **10** when a user logs into the medical device **10** in a servicing mode (since presumably the user in this case is the biomedical engineer, IT person, or other person engaged in servicing of the medical device **10).** In addition, notifications of unused security features can be added to other communication, e.g., when contacted for a particular vulnerability. The email or other communication client of the targeted customer contact receives the notification **26,** for example, as an email of targeted customer contact (e.g., basic notification or PDF report as attachment). In some examples, the information in the notification **26** can also be collected by the manufacturer and provided in response to any inquiries regarding a software bill of materials (SBOM) and Vulnerability Exploitability Exchange (VEX) for the particular imaging system **10.**

In some embodiments, the security feature usage analysis **52** and/or communication via the person-of-interest oracle **20** is dependent on pre-defined positions in the lifecycle of the medical device **10.** For example, the operation **52** may be performed after installation of the medical device **10,** after an upgrade or other modification of the medical device **10,** after onboarding new security services, at pre-defined intervals, or at other trigger times and/or events.

In some embodiments, a notification effectiveness check **58** is performed to measure effectiveness of the notification. For example, the notification effectiveness check **58** can be performed at a predetermined time after the notification **26** is sent (e.g., a few days after, a week after, a month after, et cetera) to determine whether the unused security feature has now been activated. The advertisement effectiveness check **58** suitably analyzes the log data **11** generated after the notification **26** is sent to the targeted user using the same type of analysis employed by the operation **52** to detect whether the security feature is now activated. If not, then the person of interest oracle **20** can be invoked to select another targeted customer contact (that is, a different customer contact than the person originally targeted by the notification **26).** The feedback on effectiveness of the notification **26** determined by the check **58** can also be used to dynamically update the training of the ML model **24,** for example using cases in which the notification **26** was effective (security feature activated) as further positive training examples and using cases in which the notification **26** was not effective (security feature not activated) as further negative training examples. In some examples, the feedback can analyze trends to create new templates or new risk factors.

In a corresponding method embodiment, at an operation **52,** an unused or modified security feature of the medical device **10** is detected by analysis of the log data **11.** In one embodiment, the detecting operation **52** includes analyzing user logins to the medical device **10** recorded in the log data **11** of the medical device **10** to detect the unused security feature as a strong authentication (e.g., a username/password requirement) for login to the medical device **10.** In another embodiment, the detecting operation **52** includes analyzing firewall events of the medical device **10** recorded in the log data (e.g. start/stop events of the firewall service in the system event logs, allowed/blocked network traffic in the firewall logs) **11** to detect the unused security feature as a presence of a firewall in software of the medical device **10.** In another embodiment, the detection operation **52** includes determining a lack of end-point protection software events (e.g., update/detect/block/allow events by anti-virus or software whitelisting solutions) recorded in the log data **11** to detect the unused security feature as not using an end-point protection solution to protect the medical device **10.** In another embodiment, the detecting operation **52** includes analyzing the log data **11** to determine a lack of a key or certificate recorded in the log data (i.e., in DICOM format) to detect the unused security feature as not using data encryption.

At an operation **54,** a contact is determined to whom a notification **26** of the unused security feature should be sent. To do so, a list of a plurality of contacts (e.g., the registry **22)** is analyzed, and information about each contact in the list is determined. In another example, the list of contacts can be generated by analyzing login information recorded in the log data **11** to identify the plurality of contacts as the users of the medical device **10.** A targeted contact is then selected based on the determined information.

At an operation **56,** the notification **26** is generated and to the determined targeted customer contact. The advertisement **26** includes information identifying the unused security feature. In some examples, the notification **26** is generated by populating a template notification with information specific to the determined customer contact. If the operation **52** identified multiple unused security features, then the notification **26** is suitably generated by aggregating information related to multiple detected unused security features. (Put another way, a single notification **26** may notify the targeted customer contact about the multiple unused security features).

In some embodiments, the notification effectiveness check **58** is configured to determine whether the unused security feature is implemented (e.g., used or enabled) in the medical device **10** at a preselected time after sending the notification **26.** Upon determining that the unused security feature is not implemented in the medical device **10,** the person of interest oracle **20** is invoked to select and send the notification **26** to a different customer contact. Optionally, an indication of whether the contact has been sent the notification **26** can be stored in a database for the vendor's records.

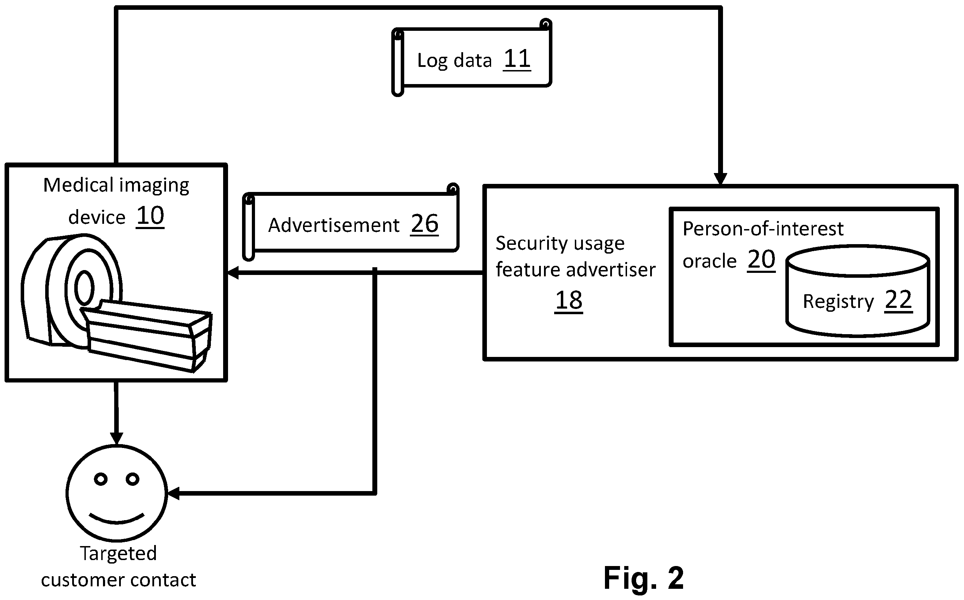

With reference to Fig. 2, another example is diagrammatically shown. In this example, the medical device is a medical imaging device **10,** e.g., an MR scanner, CT scanner, PET scanner, IGT system, or so forth. Log data **11** automatically generated by the medical imaging device **10** are sent to the security usage feature advertiser **18** implemented at the vendor's service computer, where the log data **11** are analyzed as previously described to detect one or more unused or modified security features that, if activated, would improve security of the medical imaging device **10.** The person-of-interest oracle **20** consults the customer contacts registry **22** to identify a targeted customer contact, and the notification **26** is sent from the vendor's server computer to the medical imaging device **10** (e.g. for display on the controller thereof), and/or is sent directly to the targeted customer contact (e.g., via email, text message, or the like sent to the contact's cellphone, work computer, and/or so forth).

A non-transitory storage medium includes any medium for storing or transmitting information in a form readable by a machine (e.g., a computer). For instance, a machine-readable medium includes read only memory ("ROM"), solid state drive (SSD), flash memory, or other electronic storage medium; a hard disk drive, RAID array, or other magnetic disk storage media; an optical disk or other optical storage media; or so forth.

The methods illustrated throughout the specification, may be implemented as instructions stored on a non-transitory storage medium and read and executed by a computer or other electronic processor.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

## Claims

1. A non-transitory computer readable medium **(16)** storing instructions readable and executable by at least one electronic processor **(14)** to perform a method **(100)** of promoting unused or modified security features to a customer of a medical device **(10),** the method comprising:
detecting, from log data **(11)** of the medical device, an unused or modified security feature of the medical device;
determining a contact to whom a notification **(26)** of the unused security feature should be sent; and
sending the notification to the determined contact indicative of information of the unused security feature.

2. The non-transitory computer readable medium **(16)** of claim 1, wherein the detecting includes:
analyzing user logins to the medical device **(10)** recorded in the log data **(11)** of the medical device to detect the unused security feature as a password requirement for login to the medical device.

3. The non-transitory computer readable medium **(16)** of claim 1, wherein the detecting includes:
analyzing firewall events recorded in the log data **(11)** of the medical device **(10)** to detect the unused security feature as a presence of a firewall in software of the medical device.

4. The non-transitory computer readable medium **(16)** of claim 1, wherein the detecting includes:
determining a lack of end-point protection software events recorded in the log data **(11)** of the medical device **(10)** to detect the unused security feature as not using an end-point protection solution to protect the medical device.

5. The non-transitory computer readable medium **(16)** of claim 1, wherein the detecting includes:
analyzing the log data **(11)** to determine a lack of a key or certificate recorded in the log data to detect the unused security feature as not using data encryption.

6. The non-transitory computer readable medium **(16)** of any one of claims 1-5, wherein determining a contact to whom a notification **(26)** of the unused security feature should be sent includes:
analyzing a list of a plurality of contacts;
determining information about each contact in the list; and
selecting a targeted contact based on the determined information.

7. The non-transitory computer readable medium **(16)** of claim 6, wherein the selecting of the target contact is performed by a machine-learning model **(24).**

8. The non-transitory computer readable medium **(16)** of either one of claims 6 and 7, wherein the method **(100)** further includes:
generating the list of the plurality of contacts by analyzing login information recorded in the log data **(11)** of the medical device **(10)** to identify the plurality of contacts as the users of the medical device.

9. The non-transitory computer readable medium **(16)** of any one of claims 1-8, wherein the method **(100)** further includes:
generating the notification **(26)** by populating a template notification with information specific to the determined contact.

10. The non-transitory computer readable medium **(16)** of any one of claims 1-9, wherein the method **(100)** further includes:
generating the notification **(26)** by aggregating information related to multiple detected unused security features.

11. The non-transitory computer readable medium **(16)** of any one of claims 1-10, wherein the method **(100)** further includes:
a preselected time after sending the notification **(26),** determining whether the unused security feature is implemented in the medical device **(10).**

12. The non-transitory computer readable medium **(16)** of claim 11, wherein the selecting of the preselected time is performed by a machine-learning model **(24).**

13. The non-transitory computer readable medium **(16)** of any one of claims 1-12, wherein the notification **(26)** includes risk factors and a risk type associated with the security feature that is not used.

14. The non-transitory computer readable medium **(16)** of any one of claims 1-13, wherein the method **(100)** further includes:
providing the notification **(26)** in response to any inquiries regarding a software bill of materials (SBOM) and Vulnerability Exploitability Exchange (VEX) for the particular imaging system **10.**

15. The non-transitory computer readable medium **(16)** of any one of claims 1-14, wherein the method **(100)** further includes:
providing feedback for implementing the unused or modified security feature of the medical device **(10).**
